# EUROPEAN PATENT APPLICATION

(11) **EP 1 338 340 A1**
(43) Date of publication of application: **27.08.2003**
(21) Application number: 02356087.3
(22) Date of filing: 10.05.2002
(51) Int. Cl.: B01L 3/14, B01L 7/00, B01L 11/00, G01N 33/18, G01N 1/14

(54) **Process for controlling the temperature of wastewater samples**

(30) Priority: 26.02.2002 US 83070
(71) Applicant: ISCO, Inc., Lincoln, NE 68528-1586 (US)
(72) Inventor: Fritz, Larry Lee, Lincoln, Nebraska 68516 (US); Nabity, Frederick Alan, Lincoln, Nebraska 68532 (US)
(74) Representative: Moinas, Michel

(57) **Abstract**

A wastewater sample collection system that collects wastewater samples, deposits them into sample containers, and maintains the temperature of the samples collected within a narrow temperature range. The system utilizes a microprocessor-based controller for controlling the refrigeration system and the sample collection, a sample collecting pump, a sample container, a sensor to accurately sense the temperature of the collected sample, an enclosure around the sample container, and a refrigeration system to cool the interior of the enclosure.

## Description

### BACKGROUND OF THE INVENTION

### Field of the Invention

The present invention relates to the field of wastewater sample collection systems, and more particularly to a system that maintains the temperature of the collected samples within a narrow temperature range.

### Description of Related Art

When taking samples for analysis of wastewater, it is important to maintain a low temperature so that biological and chemical processes are halted or slowed. It has been established as a standard that the sample temperature should be maintained at a temperature of no more than 4 degrees Celsius. In the sampling industry, various methods have been used including portable ice chilled samplers as well as fixed installations involving refrigeration systems.

Refrigeration systems attempting to maintain temperatures in the range of 0 to 4 degrees C have an inherent problem in that the temperature of the refrigeration coils will drop below the frost point causing frost to form. If the system does not ensure that the temperature rises above the freezing point, the frost will accumulate and degrade the performance of the system. For this reason, refrigeration systems typically use a thermostat to control the compressor that is in good thermal communication with the evaporator or cooling coils with the thermostat calibrated to ensure that the system will not start until the temperature of the evaporator is above the freezing point. This does a good job of controlling the temperature of the refrigeration system's evaporator, but the temperature of the air in the refrigeration enclosure is dependent upon not only the temperature of the evaporator, but also the thermal load due to heat entering through the enclosure skin and heat from collected samples. As the heat load increases, the temperature difference between the evaporator and the air temperature will increase.

Some time back, an improvement was made on this by introducing two temperature sensors to control the system. One sensor is located so that it measures the air temperature within the sample storage cabinet. The other temperature sensor is located so that it measures the temperature of the refrigeration system's evaporator coils. Logic then controls the system to ensure that the evaporator is sufficiently warm to melt the frost before allowing the compressor to start, while allowing the compressor to run until the air sensing sensor senses the desired temperature.

While this system does a good job of accurately controlling the air temperature within the unit over a wide range of ambient conditions, the temperature of the samples will still typically be at a higher temperature than the air due to the fact that some temperature differential is necessary to cause the heat transfer. If the system is sampling regularly (15-minute intervals) from a hot sample source (100 degrees F) it has been observed that the sample temperature will never drop below 8 to 12 degrees C even though the air temperature is maintained at 2 degrees. Of course adjusting the air temperature down would address this problem, but it would have the result of freezing the sample at the conclusion of the sampling program. Also it would be difficult to predict the temperature offset needed for every sampling program and temperature situation possible.

As a consequence of the foregoing situation, there has existed a longstanding need for a new and improved process for controlling the temperature of wastewater samples and the provision of such a system is a stated objective of the present invention.

### BRIEF SUMMARY OF THE INVENTION

Briefly stated, the present invention provides a wastewater sample collection system that collects wastewater samples, deposits them into sample containers, and maintains the temperature of the samples collected within a narrow temperature range. The system utilizes a microprocessor-based controller for controlling the refrigeration system and the sample collection, a sample collecting pump, a sample container, a sensor to accurately sense the temperature of the collected sample, an enclosure around the sample container, and a refrigeration system to cool the interior of the enclosure.

### BRIEF DESCRIPTION OF THE SEVERAL VIEWS OF THE DRAWINGS

These and other attributes of the invention will become more clear upon a thorough study of the following description of the best mode for carrying out the invention, particularly when reviewed in conjunction with the drawings, wherein:

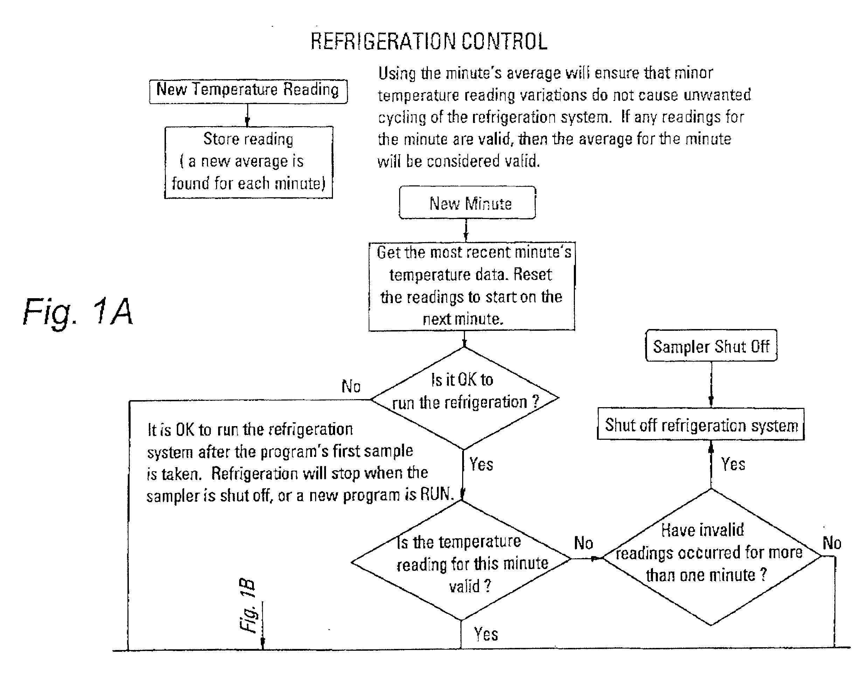

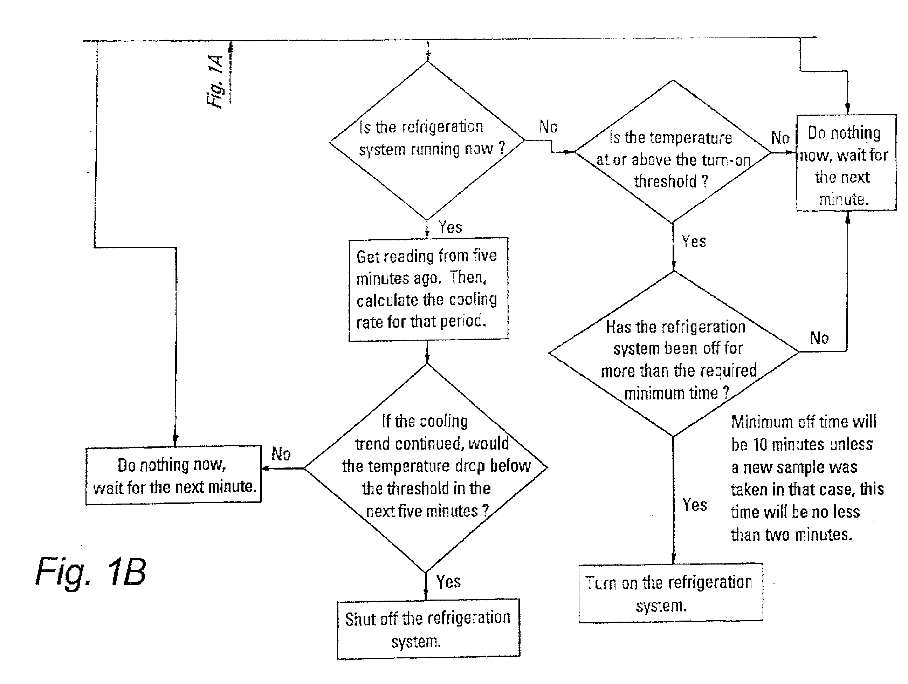

FIG. 1A and 1B are a flow chart illustrating the software algorithm used in the process of the present invention.

### DETAILED DESCRIPTION OF THE INVENTION

One embodiment of the present invention is a process that uses the basic control system described in the prior art, but with the temperature sensor that normally measures the air temperature placed directly into the sample container. The result is that once the compressor starts, it will run until the temperature of the sample is below the set point, typically 3 degrees C. In this embodiment, the sensor remains that measures the evaporator to ensure that once the compressor stops, it stays off until the frost melts.

Significant improvements in controlling the sample temperature were achieved with this system. Since the system runs the compressor until the sample is cooled to the target temperature, the air temperature in the enclosure is lowered significantly below the "nominal" 3 degrees C. This enhances the heat transfer. When the sample temperature reaches the desired temperature, the system turns the refrigeration system off, allowing the air temperature to rise to essentially the 2 to 4 degree C range. As a result, the temperature of the sample will be rapidly brought down to the target temperature, while eliminating any possibility of freezing the sample.

A second embodiment is a variation on the system of the first embodiment with the following differences. The second embodiment utilizes a small icebox sized refrigerator of dimensions about 26 x 15 x 20 inches, whereas the first embodiment uses a much larger case. The first embodiment uses 2 temperature sensors, one to sense the temperature of the refrigeration systems evaporator, the other to sense the collected sample temperature. The second embodiment only uses one temperature sensor. The sensors used in the first embodiment are capable of generating only a binary signal indicating that the temperature was over/under a set temperature. The second system uses a temperature sensor that is capable of outputting a digital signal of the temperature with resolution to 0.1 degree C. By using a smart controller for the refrigeration system based upon the measured temperature of the collected sample, frosting of the evaporator can be controlled without the need for a separate sensor on the evaporator. Using the smart controller, starting the refrigeration system too quickly after shutting off (short cycling) is prevented by incorporating a timer to set a minimum off time. Algorithms can be developed to minimize the temperature overshoot that occurs when the sample continues to get colder even after the refrigeration system is shut off. This is particularly a problem in applications where the outside (ambient) air temperature is low. It is anticipated that the collected sample can be kept from freezing even if the ambient temperature is -10 degrees C.

The sample temperature measurement sensor may alternately be of a direct measurement type such as a thermistor, thermocouple, RTD, etc. directly placed within the sample collection container, or an indirect, non-contact measurement type such as an infrared temperature measurement system.

The control algorithm, in order to minimize power consumption, leaves the refrigeration system "off" until the conditions are met for the collection of the first sample.

The basic control algorithm runs the compressor when the temperature of the collected sample is higher than the set point temperature and stops the compressor when the temperature is less than the set point temperature, given that once the compressor is stopped, it remains off a minimum off time which is a parameter of the specific refrigeration system, for example, a minimum off time of 15 seconds.

Also incorporated into the control is an algorithm that turns the refrigeration system off before the desired temperature is reached so that the temperature does not overshoot the desired temperature after the compressor is stopped.

As shown in FIG. 1A and 1B, the essence of the algorithm that compensates for anticipated temperature changes is as follows: On each evaluation cycle (which occurs at one minute intervals) the system calculates the temperature rate of change in the last 5 minutes. Assuming that the temperature rate of change continues, the system calculates a predicted temperature after the next five minutes. If that temperature is below the set point (3 degrees in our embodiment), the system shuts the compressor off. Once off, the system will remain off for a minimum of 10 minutes unless a new sample is taken.

Although only an exemplary embodiment of the invention has been described in detail above, those skilled in the art will readily appreciate that many modifications are possible without materially departing from the novel teachings and advantages of this invention. Accordingly, all such modifications are intended to be included within the scope of this invention as defined in the following claims.

## Claims

1. A process for controlling the temperature of wastewater samples, comprising the steps of:
collecting a wastewater sample from a wastewater stream into a sample container;
placing the sample container in a refrigerated enclosure cooled by selective operation of a compressor;
disposing a temperature sensor within the enclosure to measure the temperature of the collected sample; and
controlling the operation of the compressor based on the measured temperature of the collected sample.

2. The process of claim 1 wherein the temperature sensor is positioned within the sample container.

3. The process of claim 2 wherein the temperature sensor is a direct measurement sensor.

4. The process of claim 3 wherein the temperature sensor is a thermistor.

5. The process of claim 3 wherein the temperature sensor is a thermocouple.

6. The process of claim 3 wherein the temperature sensor is a RTD.

7. The process of claim 1 wherein the temperature sensor is a non-contact sensor spaced from the sample container.

8. The process of claim 7 wherein the temperature sensor is an infrared sensor.

9. The process of claim 1 wherein the temperature sensor outputs a digital signal of the temperature.

10. The process of claim 9 wherein the digital signal of the temperature has a resolution to 0.1 degrees C.

11. The process of claim 1 wherein the temperature sensor outputs a binary signal indicating the temperature.

12. The process of claim 1 further including the step of controlling the operation at the compressor based on the rate of change of the temperature of the collected sample.
